(19) Europäisches Patentamt
European Patent Office
Office européen des brevets

(11) Veröffentlichungsnummer: **0 319 791 B1**

(12) **EUROPÄISCHE PATENTSCHRIFT**

(45) Veröffentlichungstag der Patentschrift: **22.07.92**

(51) Int. Cl.5: **C07D 405/10**, A01N 43/38

(21) Anmeldenummer: **88119633.1**

(22) Anmeldetag: **25.11.88**

Die Akte enthält technische Angaben, die nach dem Eingang der Anmeldung eingereicht wurden und die nicht in dieser Patentschrift enthalten sind.

(54) **N-Phenyltetrahydrophthalimide.**

(30) Priorität: **05.12.87 DE 3741272**

(43) Veröffentlichungstag der Anmeldung:
**14.06.89 Patentblatt 89/24**

(45) Bekanntmachung des Hinweises auf die Patenterteilung:
**22.07.92 Patentblatt 92/30**

(84) Benannte Vertragsstaaten:
**CH DE FR GB IT LI**

(56) Entgegenhaltungen:
**EP-A- 0 207 894**
**ZA-A- 8 703 933**

(73) Patentinhaber: **BASF Aktiengesellschaft**
**Carl-Bosch-Strasse 38**
**W-6700 Ludwigshafen(DE)**

(72) Erfinder: **Rueb, Lothar, Dr.**
**Zum Weidentor 4**
**W-6720 Speyer(DE)**
Erfinder: **Eicken, Karl, Dr.**
**Am Huettenwingert 12**
**W-6706 Wachenheim(DE)**
Erfinder: **Pander, Hans Joachim**
**Hochdorfer Strasse 19**
**W-6701 Roedersheim-Gronau(DE)**
Erfinder: **Plath, Peter, Dr.**
**Hans-Balcke-Strasse 13**
**W-6710 Frankenthal(DE)**
Erfinder: **Schwalge, Barbara, Dr.**
**Adolf-Diesterweg-Strasse 77**
**W-6700 Ludwigshafen(DE)**
Erfinder: **Wuerzer, Bruno, Dr.**
**Ruedigerstrasse 13**
**W-6701 Otterstadt(DE)**
Erfinder: **Meyer, Norbert, Dr.**
**Dossenheimer Weg 22**
**W-6802 Ladenburg(DE)**

**EP 0 319 791 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es sind bereits N-arylsubstituierte Tetrahydrophthalimide mit Herbizidwirkung bekannt. z.B. werden in der EP 207 894 u.a. Tetrahydrophthalimide der Formel I′ beschrieben,

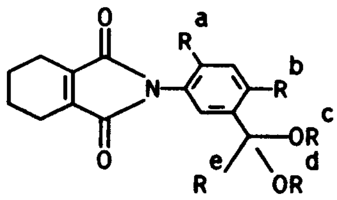

I′

in der Reste beispielsweise folgende Bedeutung haben:

$R^a$ und $R^b$ unabhängig voneinander Wasserstoff oder Halogenatom
$R^c$ und $R^d$ unter anderem gemeinsam eine gegebenenfalls substituierte $C_2$-$C_3$-Alkylenbrücke und
$R^e$ unter anderem Wasserstoff, Cyanogruppe oder $C_1$-$C_4$-Alkylgruppe.

Diese Verbindungen zeigen jedoch bei geringen Aufwandmengen eine unbefriedigende Wirksamkeit.

Der Erfindung lag daher die Aufgabe zugrunde, N-Phenyltetrahydrophthalimidverbindungen zu finden, die bei geringer Aufwandmenge eine bessere Wirksamkeit gegen unerwünschte Pflanzen zeigen, ohne die Kulturpflanzen zu schädigen.

Entsprechen dieser Aufgabe wurde gefunden, daß Tetrahydrophthalimide der allgemeinen Formel I

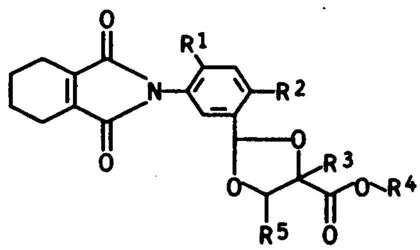

in der bedeutet:

$R^1$ Wasserstoff oder Halogen
$R^2$ Halogen
$R^3$ Wasserstoff oder $C_1$-$C_4$-Alkyl
$R^4$ unsubstituiertes oder durch $C_1$-$C_3$-Alkoxy substituiertes $C_1$-$C_8$-Alkyl, $C_3$-$C_4$-Alkenyl oder $C_3$-$C_4$-Alkinyl
$R^5$ Wasserstoff oder $C_1$-$C_4$-Alkyl

eine vorteilhafte herbizide Wirkung, besonders im Nachauflaufverfahren haben und gegenüber einer Reihe von Kulturpflanzen selektiv sind.

Unter Halogen wie vorstehend verwendet, ist Fluor, Chlor und Brom zu verstehen, wobei $R^1$ in der Bedeutung für Halogen bevorzugt für Fluor und $R^2$ bevorzugt für Chlor steht.

Die Bezeichnung Alkyl umfaßt verzweigte und geradkettige Reste, z.B. Methyl, Ethyl, n-Propyl und Isopropyl.

Die Alkenyl- und Alkinylreste können ebenfalls verzweigt oder geradkettig sein. Beispiele für Alkenylreste in Formel I sind 2-Propenyl, 2-Butenyl, 3-Butenyl, 2-Isobutenyl, insbesondere 2-Propenyl oder 2-Butenyl, Alkinylreste sind in der Regel Propargyl, 2-Butinyl und 3-Butinyl.

Die Erfindung umfaßt die Enantiomeren, die Diastereomeren und deren Gemische.

Die N-substituierten Tetrahydrophthalimide sind aus 3.4.5.6-Tetrahydrophthalsäureanhydrid und einem Anilin der Formel V, z.B. in einem Lösungsmittel bei einer Temperatur von 20 bis 200 °C, vorzugsweise 40 bis 150 °C, erhältlich. Als Lösungsmittel eignen sich z.B. niedere Alkansäuren wie Eisessig oder Propionsäure oder aprotische Lösungsmittel wie Toluol oder Xylol in Gegenwart von sauren Katalysatoren wie aromatischen Sulfonsäuren.

Unter den Verbindungen I sind diejenigen bevorzugt, in denen $R^1$ Wasserstoff oder Fluor, $R^2$ Chlor, $R^3$ $C_1$-$C_2$-Alkyl, $R^4$ $C_1$-$C_4$-Alkyl, $C_3$-$C_4$-Alkenyl oder $C_3$-$C_4$-Alkinyl und $R^5$ Wasserstoff oder Methyl bedeutet.

Die Aniline der Formel V können z.B. dadurch erhalten werden, daß man ein Nitrobenzol IV entsprechender Acetalstruktur durch Reduktionsmittel wie z.B. Eisen oder Zinn(II)Salze reduziert.

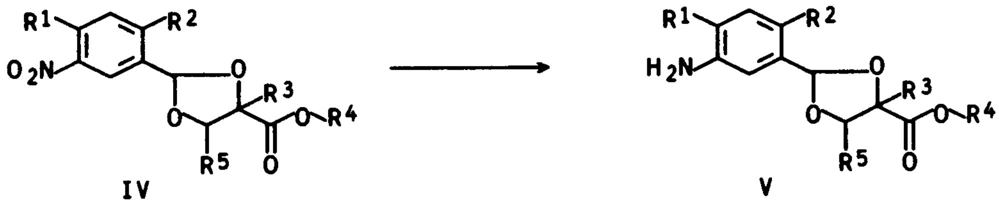

IV V

Die Reduktion kann auch als katalytische Hydrierung mit einem Edelmetallkatalysator wie Platin oder Palladium oder mit Raney-Nickel bei relativ milden Bedingungen durchgeführt werden.

Nitroacetale IV sind durch Umsetzung eines entsprechenden Benzaldehyds II in einem inerten Lösungsmittel in Gegenwart einer Säure mit einem Diol der Formel III zugänglich.

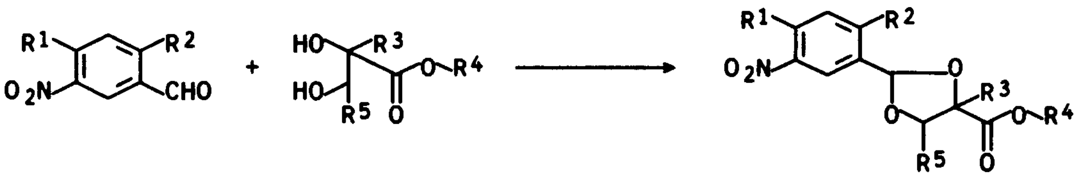

II III IV

Die in den nachstehenden Beispielen wiedergegebenen Arbeitsempfehlungen wurden unter entsprechender Abwandlung der Ausgangsverbindungen zur Gewinnung weiterer Verbindungen der allgemeinen Formel I benutzt. Die Verbindungen sind mit physikalischen Daten in der folgenden Tabelle aufgeführt. Verbindungen ohne solche Angaben lassen sich aus entsprechenden Stoffen in analoger Weise erhalten. Sie lassen aufgrund ihrer nahen strukturellen Beziehung zu den hergestellten und untersuchten Verbindungen eine gleichartige Wirkung erwarten.

Beispiel

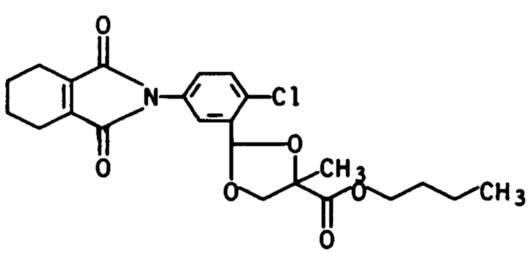

a) Zu 18,6 g 2-Chlor-5-nitro-benzaldehyd und 0,5 g p-Toluolsulfonsäure in 250 ml Toluol werden 19,4 g 2.3-Dioxyisobuttersäure-n-butylester gegeben und 5 Stunden unter Rückfluß am Wasserabscheider gekocht. Nach dem Abkühlen wird das Lösungsmittel entfernt und im Hochvakuum getrocknet. Man erhält 35 g 3-(5-Methyl-5-n-butyloxycarbonyl-1.3-dioxolan-2-yl)-4-chlor-nitrobenzol (öl).

b) Zu einer Mischung von 16,8 g Eisenpulver in 30 ml Methanol und 75 ml Eisessig gibt man bei Rückfluß 34,4 g der obigen Nitroverbindung in 20 ml Methanol und erhitzt 2 Stunden am Rückfluß. Nach dem Abkühlen werden 250 ml Wasser zugegeben. und abgesaugt. Das Filtrat wird 3 mal mit je 100 ml Essigester extrahiert, getrocknet, eingeengt und im Hochvakuum getrocknet. Man erhält 31 g 3-(5-Methyl-5-n-butyloxycarbonyl-1.3-dioxolan-2-yl)-4-chlor- anilin (öl).

c) 15,7 g des obigen Anilins und 7,6 g Cyclohexen-1.2-dicarbonsäureanhydrid werden in 150 ml Eisessig 5 Stunden unter Rückfluß erhitzt. Nach dem Abkühlen gibt man 150 ml Wasser zu und extrahiert 2 mal mit je 100 ml Methylenchlorid, trocknet und entfernt das Lösungsmittel. Das Produkt wird durch

Chromatographie gereinigt und im Hochvakuum getrocknet. Man erhält 9,0 g N-[3-(5-Methyl-5-n-butyloxycarbonyl-1.3-dioxolan-2-yl)-4-chlor-phenyl]-3.4.5.6-tetrahydrophthalimid (öl) (Tabelle 1 Nr. 1.027).

Weitere Beispiele für Wirkstoffe, die nach diesem Syntheseprinzip hergestellt werden können, finden sich in Tabelle 1.

Tabelle 1

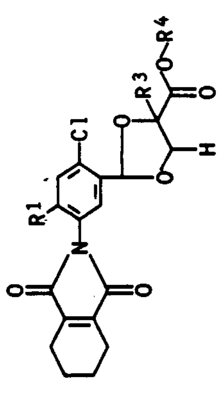

| Nr. | $R^1$ | $R^3$ | $R^4$ | Fp [°C] | IR [$cm^{-1}$] |
|---|---|---|---|---|---|
| 1.001 | H | H | $CH_3$ | | |
| 1.002 | F | H | $CH_3$ | | |
| 1.003 | H | $CH_3$ | $CH_3$ | öl | 1715, 1481, 1377, 1088 |
| 1.004 | F | $CH_3$ | $CH_3$ | | |
| 1.005 | H | $CH_2CH_3$ | $CH_3$ | öl | 2938, 1715, 1481, 1378, 1088 |
| 1.006 | F | $CH_2CH_3$ | $CH_3$ | | |
| 1.007 | H | H | $CH_2CH_3$ | | |
| 1.008 | F | H | $CH_2CH_3$ | | |
| 1.009 | H | $CH_3$ | $CH_2CH_3$ | öl | 1715, 1480, 1376, 1088 |
| 1.010 | F | $CH_3$ | $CH_2CH_3$ | öl | 1720, 1500, 1422, 1082 |
| 1.011 | H | $CH_2CH_3$ | $CH_2CH_3$ | öl | 2938, 1715, 1480, 1377, 1089 |
| 1.012 | F | $CH_2CH_3$ | $CH_2CH_3$ | öl | 1721, 1500, 1422, 1083 |
| 1.013 | H | H | $(CH_2)_2CH_3$ | | |
| 1.014 | F | H | $(CH_2)_2CH_3$ | | |
| 1.015 | H | $CH_3$ | $(CH_2)_2CH_3$ | öl | 2929, 1716, 1479, 1377, 1089 |
| 1.016 | F | $CH_3$ | $(CH_2)_2CH_3$ | öl | 1720, 1500, 1422, 1082 |
| 1.017 | H | $CH_2CH_3$ | $(CH_2)_2CH_3$ | öl | 2938, 1716, 1481, 1378, 1089 |
| 1.018 | F | $CH_2CH_3$ | $(CH_2)_2CH_3$ | öl | 1721, 1500, 1422, 1083 |

Tabelle 1 (Fortsetzung)

| Nr. | $R^1$ | $R^3$ | $R^4$ | Fp [°C] | IR [$cm^{-1}$] |
|---|---|---|---|---|---|
| 1.019 | H | H | $CH(CH_3)_2$ | | |
| 1.020 | F | H | $CH(CH_3)_2$ | | |
| 1.021 | H | $CH_3$ | $CH(CH_3)_2$ | | |
| 1.022 | F | $CH_3$ | $CH(CH_3)_2$ | | |
| 1.023 | H | $CH_2CH_3$ | $CH(CH_3)_2$ | | |
| 1.024 | F | $CH_2CH_3$ | $CH(CH_3)_2$ | | |
| 1.025 | H | H | $(CH_2)_3CH_3$ | | |
| 1.026 | F | H | $(CH_2)_3CH_3$ | | |
| 1.027 | H | $CH_3$ | $(CH_2)_3CH_3$ | Öl | 2958, 1716, 1481, 1377, 1088 |
| 1.028 | F | $CH_3$ | $(CH_2)_3CH_3$ | Öl | 2958, 1721, 1500, 1423, 1082 |
| 1.029 | H | $CH_2CH_3$ | $(CH_2)_3CH_3$ | Öl | 2960, 1716, 1480, 1378, 1089 |
| 1.030 | F | $CH_2CH_3$ | $(CH_2)_3CH_3$ | Öl | 2960, 1721, 1500, 1422 |
| 1.031 | H | $CH_3$ | $CH_2CH(CH_3)_2$ | Öl | 2960, 1718, 1481, 1378, 1088 |
| 1.032 | F | $CH_3$ | $CH_2CH(CH_3)_2$ | | |
| 1.033 | H | $CH_2CH_3$ | $CH_2CH(CH_2CH_3)(CH_2)_3CH_3$ | Öl | 2932, 1717, 1481, 1378, 1089 |
| 1.034 | F | $CH_2CH_3$ | $CH_2CH(CH_2CH_3)(CH_2)_3CH_3$ | | |

Tabelle 2

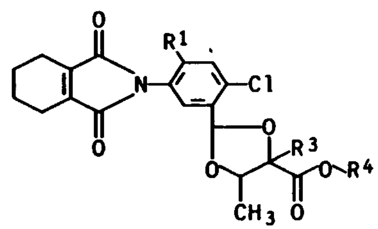

| Nr. | $R^1$ | $R^3$ | $R^4$ | Fp [°C] | IR [$cm^{-1}$] |
|---|---|---|---|---|---|
| 2.001 | H | $CH_3$ | $CH_3$ | | |
| 2.002 | F | $CH_3$ | $CH_3$ | | |
| 2.003 | H | $CH_3$ | $CH_2CH_3$ | | |
| 2.004 | F | $CH_3$ | $CH_2CH_3$ | | |
| 2.005 | H | $CH_3$ | $(CH_2)_2CH_3$ | | |
| 2.006 | F | $CH_3$ | $(CH_2)_2CH_3$ | | |
| 2.007 | H | H | $CH(CH_3)_2$ | Öl | 2936, 1717, 1418, 1376, 1101 |
| 2.008 | F | H | $CH(CH_3)_2$ | | |
| 2.009 | H | H | $(CH_2)_3CH_3$ | Öl | 2958, 1715, 1480, 1378, 1100 |
| 2.010 | F | H | $(CH_2)_3CH_3$ | | |
| 2.011 | H | H | $CH_3$ | Öl | 1715, 1481, 1378, 1104 |
| 2.012 | F | H | $CH_3$ | | |
| 2.013 | H | H | $(CH_2)_2CH_3$ | | |
| 2.014 | F | H | $(CH_2)_2CH_3$ | | |

Tabelle 3

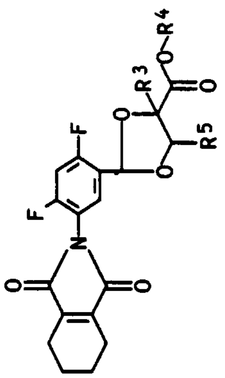

| Nr. | R3 | R4 | R5 | Fp [°C] | IR [$cm^{-1}$] |
|---|---|---|---|---|---|
| 3.001 | H | $CH_3$ | H | | |
| 3.002 | $CH_3$ | $CH_3$ | H | | |
| 3.003 | H | $CH_3$ | $CH_3$ | | |
| 3.004 | $CH_3$ | $CH_3$ | $CH_3$ | | |
| 3.005 | H | $CH_2CH_3$ | H | | |
| 3.006 | $CH_3$ | $CH_2CH_3$ | H | | |
| 3.007 | H | $CH_2CH_3$ | $CH_3$ | | |
| 3.008 | $CH_3$ | $CH_2CH_3$ | $CH_3$ | | |
| 3.009 | H | $(CH_2)_2CH_3$ | H | | |
| 3.010 | $CH_3$ | $(CH_2)_2CH_3$ | $CH_3$ | | |
| 3.011 | H | $(CH_2)_2CH_3$ | $CH_3$ | | |
| 3.012 | $CH_3$ | $(CH_2)_2CH_3$ | $CH_3$ | | |
| 3.013 | H | $CH(CH_3)_2$ | H | | |
| 3.014 | $CH_3$ | $CH(CH_3)_2$ | H | | |
| 3.015 | H | $CH(CH_3)_2$ | $CH_3$ | öl | 2978, 1720, 1512, 1437, 1097, 1068 |
| 3.016 | $CH_3$ | $CH(CH_3)_2$ | $CH_3$ | | |
| 3.017 | H | $(CH_2)_3CH_3$ | H | | |
| 3.018 | $CH_3$ | $(CH_2)_3CH_3$ | H | öl | 1720, 1512, 1437, 1132 |
| 3.019 | H | $(CH_2)_3CH_3$ | $CH_3$ | | |
| 3.020 | $CH_3$ | $(CH_2)_3CH_3$ | $CH_3$ | | |

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,005 bis 3,0 vorzugsweise 0,01 bis 0,5 kg/ha.

Die herbizide Wirkung der Tetrahydroisoindoldionderivate der Formel I auf das Wachstum der Testpflanzen wird durch folgende Gewächshausversuche gezeigt.

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt eingesät.

Zum Zweck der Nachauflaufbehandlung werden entweder direkt gesäte oder in den gleichen Gefäßen aufgewachsene Pflanzen ausgewählt oder sie werden erst als Keimpflanzen getrennt angezogen und einige Tage vor der Behandlung in die Versuchsgefäße verpflanzt.

Je nach Wuchsform werden die Testpflanzen bei einer Wuchshöhe von 3 bis 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,06 kg/ha a.S.

Die Versuchsgefäße werden im Gewächshaus aufgestellt, wobei für wärmeliebende Arten wärmere Bereiche (20 bis 35 ° C) und für solche gemäßigter Klimate 10 bis 20 ° C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen gehören den folgenden Arten an:

| CODE | Lateinischer Name | Deutscher Name | Englischer Name |
|---|---|---|---|
| ABUTH | Abutilon theophrasti | Chinesischer Hanf | velvet leaf |
| AMARE | Amaranthus spp. | Fuchsschwanzarten | pigweed |
| CHYCO | Chrysanthemum corinarium | Kronenwucherblume | crown daisy |
| DEDTO | Desmodium tortuosum | - | Florida beggarweed |
| GALAP | Galium aparine | Klettenlabkraut | catchweed bedstraw |
| IPOSS | Ipomoea spp. | Prunkwindearten | morningglory |
| LAMAM | Lamium amplexicaule | Stengelumfassende Taubnessel | henbit |
| MERAN | Mercurialis annua | Einjähriges Bingelkraut | annual mercury |
| POLPE | Polygonum persicaria | Flohknöterich | ladystumb |
| SINAL | Sinapis alba | Weißer Senf | white mustard |
| SOLNI | Solanum nigrum | Schwarzer Nachtschatten | black nightshade |
| STEME | Stellaria media | Vogelsternmiere | chickweed |
| TRZAS | Triticum aestivum | Sommerweizen | wheat |
| TRZAW | Triticum aestivum | Weizen | wheat |
| VERSS | Veronica spp. | Ehrenpreisarten | speedwell |
| ZEAMX | Zea mays | Mais | Indian corn |

In Anbetracht der Vielseitigkeit der Applikationmethoden können die erfindungsgemäßen Verbindungen bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napobrassica | Kohlrübe |
| Brassica napus var. rapa | Weiße Rübe |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |

| Botanischer Name | Deutscher Name |
|---|---|
| Citrus reticulata | Mandarine |
| Citrus sinensis | Apfelsine, Orange |
| Coffea arabica (C. canephora, C. liberica) | Kaffee |
| Cucumis melo | Melone |
| Cynodon dactylon | Bermudagras |
| Elaeis guineensis | Ölpalme |
| Fragaria vesca | Erdbeere |
| Glycine max | Sojabohne |
| Gossypium hirsutum (G. arboreum, Gossypium herbaceum G. vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |

| Botanischer Name | Deutscher Name |
|---|---|
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (S. vulgare) | Mohrenhirse |
| Sorghum dochna | Zuckerhirse |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die N-Phenyltetrahydrophthalimide der Formel I sowohl unter sich als auch mit zahlreichen Vertretern anderer herbizider oder wachstumsregulierender Wirkstoffgruppen gemischt und gemeinsam ausgeberacht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate und andere in Betracht.

Außerdem kann es von Nutzen sein, die N-Phenyltetrahydrophthalimide der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Tabelle 4

| Herbizide Wirkung von Beispiel 1.027 bei Nachauflaufanwendung von 0,06 kg/ha im Gewächshaus | |
|---|---|
| Testpflanzen | Schädigung (%) |
| ABUTH | 100 |
| AMARE | 100 |
| DEDTO | 100 |
| GALAP | 98 |
| IPOSS | 100 |
| MERAN | 100 |
| SOLNI | 100 |

Tabelle 5

| Herbizide Wirkung und Verträglichkeit für eine Beispielkultur bei Nachauflaufanwendung von Beispiel 1.027 mit 0,03 kg/ha | |
|---|---|
| Testpflanzen | Schädigung (%) |
| GALAP | 100 |
| LAMAM | 100 |
| MERAN | 100 |
| POLPE | 100 |
| SINAL | 95 |
| STEME | 100 |
| TRZAW | 0 |
| VERSS | 100 |

Beispiele zur Bekämpfung unerwünschter breitblättriger Pflanzen und Verträglichkeit für eine Beispielkultur bei Nachauflaufapplikation von 0,03 kg/ha a.S. im Gewächshaus.

Tabelle 6

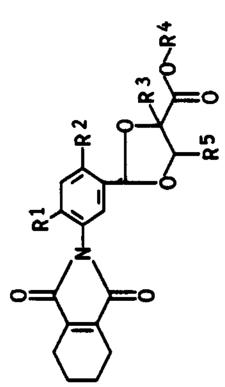

| Bsp.-Nr. | R1 | R2 | R3 | R4 | R5 | TRZAS | CHYCO | SOLNI | GALAP | STEME |
|---|---|---|---|---|---|---|---|---|---|---|
| 2.007 | H | Cl | H | $CH(CH_3)_2$ | $CH_3$ | 10 | 100 | 100 | 100 | 90 |
| 2.009 | H | Cl | H | $(CH_2)_3CH_3$ | $CH_3$ | 10 | 100 | 100 | 100 | 100 |

Tabelle 7

| Bsp.-Nr. | R1 | R2 | R3 | R4 | R5 | ZEAMX | CHYCO | LAMAM | SOLNI | STEME |
|---|---|---|---|---|---|---|---|---|---|---|
| 1.031 | H | Cl | $CH_3$ | $CH_2CH(CH_3)_2$ | H | 0 | 100 | 100 | 100 | 100 |

Tabelle 8

| Bsp.-Nr. | R1 | R2 | R3 | R4 | R5 | TRZAS | CHYCO | SOLNI | VERSS |
|---|---|---|---|---|---|---|---|---|---|
| 1.005 | H | Cl | $CH_2CH_3$ | $CH_3$ | H | 10 | 100 | 100 | 100 |

## Patentansprüche

**1.** N-Phenyltetrahydrophthalimid der allgemeinen Formel I

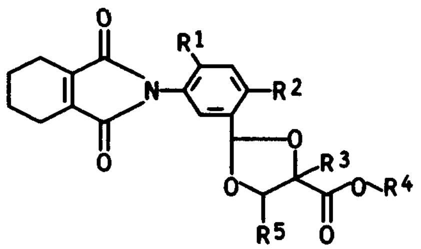

in der bedeutet:

- $R^1$ Wasserstoff oder Halogen
- $R^2$ Halogen
- $R^3$ Wasserstoff oder $C_1$-$C_4$-Alkyl
- $R^4$ unsubstituiertes oder durch $C_1$-$C_3$-Alkoxy substituiertes $C_1$-$C_8$-Alkyl, $C_3$-$C_4$-Alkenyl oder $C_3$-$C_4$-Alkinyl.
- $R^5$ Wasserstoff oder $C_1$-$C_4$-Alkyl,

unabhängig von der jeweiligen sterischen Anordnung.

**2.** Verfahren zur Herstellung von Verbindungen der Formel I gemäß Anspruch 1, dadurch gekennzeichnet, daß man einen entsprechenden Nitrobenzaldehyd der Formel II

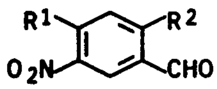

II

mit einem entsprechenden Diol der Formel III

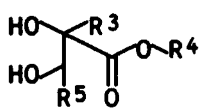

III

umsetzt, das Umsetzungsprodukt zum Anilin reduziert und mit Tetrahydrophthalsäureanhydrid umsetzt.

**3.** Herbizid wirkendes Mittel, enthaltend eine Verbindung der Formel I gemäß Anspruch 1 und übliche Hilfsstoffe, Streck- und Verdünnungsmittel.

**4.** Verwendung einer Verbindung der Formel I gemäß Anspruch 1 als Herbizid.

**5.** Verfahren zur Bekämpfung unerwünschten Pflanzenwuchses, dadurch gekennzeichnet, daß man ein Herbizid oder Mittel nach Anspruch 1 bzw. 3 auf die Pflanzen und/oder deren Lebensraum einwirken läßt.

## Claims

**1.** An N-phenyltetrahydrophthalimide of the general formula I

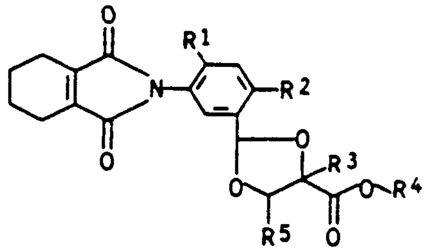

where $R^1$ is hydrogen or halogen, $R^2$ is halogen, $R^3$ is hydrogen or $C_1$-$C_4$-alkyl, $R^4$ is unsubstituted or $C_1$-$C_3$-alkoxy-substituted $C_1$-$C_8$-alkyl, $C_3$-$C_4$-alkenyl or $C_3$-$C_4$-alkynyl, and $R^5$ is hydrogen or $C_1$-$C_4$-alkyl, independently of the steric configuration in each case.

**2.** A process for the preparation of compounds of the formula I as in claim 1, wherein a corresponding nitrobenzaldehyde of the formula II

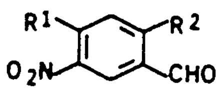

II

is reacted with a corresponding diol of the formula III

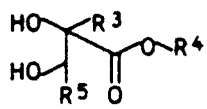

III

the reaction product is reduced to the aniline and reacted with tetrahydrophthalic anhydride.

**3.** A herbicidal agent containing a compound of the formula I as claimed in claim 1, and conventional auxiliaries, extenders and diluents.

**4.** The use of a compound of the formula I as claimed in claim I as a herbicide.

**5.** A method for controlling the growth of undesirable plants, wherein a herbicide or agent as claimed in claim 1 or 3 is allowed to act on the plants and/or their habitat.

**Revendications**

**1.** N-phényltétrahydrophtalimide de formule générale I

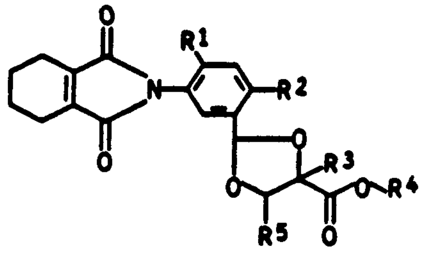

dans laquelle

$R^1$ représente hydrogène ou halogène

$R^2$, hydrogène

$R^3$, hydrogène ou alkyle en C1-C4,

$R^4$, alkyle en C1-C8, alcényle en C3-C4 ou alcynyle en C3-C4, non substitués ou substitués par alcoxy en C1-C3,

$R^5$, hydrogène ou alkyle en C1-C4,

indépendamment de l'arrangement stérique respectif.

2. Procédé de préparation de composés de formule I, selon la revendication 1, caractérisé par le fait que l'on fait réagir un nitrobenzaldéhyde approprié de formule II

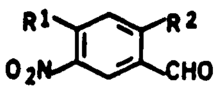

II

avec un diol approprié de formule III

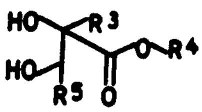

III

on réduit le produit de réaction en aniline et on le met à réagir avec de l'anhydride tétrahydrophtalique.

3. Agent à effet herbicide, contenant un composé de formule I, selon la revendication 1, et des auxiliaires, charges et diluants usuels.

4. Utilisation d'un composé de formule I, selon la revendication 1, comme herbicide.

5. Procédé de lutte contre la croissance de plantes indésirables, caractérisé par le fait que l'on fait agir sur les plantes et/ou leur biotope un herbicide ou agent selon les revendications 1 ou 3.